# EUROPEAN PATENT SPECIFICATION

(11) **EP 3 283 021 B1**
(45) Date of publication and mention of the grant of the patent: **13.03.2019**
(21) Application number: 16710003.1
(22) Date of filing: 28.01.2016
(51) Int. Cl.: A61F 5/01

(54) **ORTHOPAEDIC DEVICE FOR HALLUX VALGUS**
ORTHOPÄDISCHE VORRICHTUNG FÜR HALLUX VALGUS
DISPOSITIF ORTHOPÉDIQUE POUR L'HALLUX VALGUS

(30) Priority: 14.04.2015 IT UB20150484
(43) Date of publication of application: 21.02.2018
(73) Proprietor: Eumedica S.r.l., 35027 Noventa Padovana (PD) (IT)
(72) Inventor: DALLA FAVERA, Gianluigi, 35027 Noventa Padovana (PD) (IT); DALLA FAVERA, Susanna, 35027 Noventa Padovana (PD) (IT)
(74) Representative: Vinci, Marcello
(86) International application number: PCT/IB2016/050424
(87) International publication number: WO 2016/166614

(56) References cited:
- EP-A1- 2 524 675
- EP-A2- 1 688 106
- DE-A1-102008 049 854
- DE-A1-102011 051 083

## Description

The present invention concerns orthopaedic devices for the treatment of hallux valgus, and in particular it concerns a new improved orthopaedic device for the preventive and/or corrective and/or post-surgery treatment of hallux valgus.

The foot disease commonly known as hallux valgus consists in the lateral deviation of the big toe (hallux), with medial deviation of the first metatarsal bone.

This disease substantially leads the head of the first metatarsal bone to move away from the others, with the consequence that the forefoot opens as a fan, thus producing the characteristic bunion at the level of the metatarsal phalangeal joint and causing localized foot pain, risk of dislocation, as well as repercussions on the entire postural alignment.

Further consequences of hallux valgus are, in fact, misalignments and defects in the patient's posture, especially affecting the knees, hips and lumbar part of the spine (lower back).

Furthermore, the patients suffering from this disease cannot wear normal shoes, as the metatarsal bunion impacts against the shoe, thus causing also the formation of annoying bursitis.

Several surgical techniques for the treatment of hallux valgus are known, which are mainly intended to realign the joint of the first ray by means of a lateral translation of the head of the first metatarsal bone.

Taping techniques are also known, which are often used for the preventive and/or post-surgery therapeutic treatment of hallux valgus and are suited to provide partial immobilization without preventing the movement of the joints, but counteracting the forces that may cause undesired deviations.

The therapeutic effectiveness of taping substantially depends on correct application, according to special techniques requiring the intervention of specialized personnel, as patients cannot do it alone.

Document EP1688106 is considered to represent the closest prior art and concerns an orthopaedic device for the correction of anomalous positions of the toes, comprising a composite bar suited to be applied to the medial side of the foot, in such a way as to exert a corrective force on the big toe in the lateral direction. Said bar is made up of two pieces, including a first front branch and a second rear branch, hinged to each other through an annular hinge suited to be positioned at the level of the big toe joint.

Said bar acts on the big toe joint and thus allows the flexion/extension movement of the big toe.

The devices of this type are often uncomfortable to wear, in particular due to the thickness of the annular hinge that makes it difficult to wear normal trainers and even impossible to wear other shoes different from sports shoes.

Therefore, devices of this type are particularly suited to be used mainly at night or, however, when the user does not need to wear normal shoes.

In addition to the above, the devices of this type are made in at least two pieces and must be assembled together, which means increasing production costs and giving origin to fragile points that may easily break.

Furthermore, said annular hinge is made of a substantially rigid plastic material which presses against the big toe joint, at the level of which there may be the wound left by surgery, thus causing discomfort and even pain and consequently requiring the use of interposed pads.

Articulated devices are also known which are made up of three rigid parts hinged together, a front branch, a central branch suited to be positioned at the level of the joint and a rear branch.

The articulated devices of the known type described above must be applied very carefully, as the branches must be perfectly aligned with one another and with the foot, while the hinge needs to be correctly positioned at the level of the joint, in such a way as to allow the correct flexion-extension movement of the big toe.

In order to overcome said drawbacks, a new type of orthopaedic device for the preventive and/or corrective and/or post-surgery treatment of hallux valgus has been designed and produced.

The main object of the present invention is to provide a device that can be applied in a rapid and precise manner, even by inexpert persons or users.

It is another object of the present invention to provide a device that is comfortable, since its thickness is limited, especially at the level of or in proximity to the big toe joint.

It is another object of the present invention to provide a device that is suited to be used day and night.

It is another object of the present invention to provide a device that can be adapted to the size and shape of the user's foot.

It is another object of the present invention to provide a device whose stiffness can be adjusted according to the specific needs of the user.

It is another object of the present invention to provide a device that can be used after surgery, that is, in the presence of a wound resulting from surgery, as well as for containment, preventive and corrective treatments, especially in case of slight deviations.

It is another object of the present invention to provide a device with reduced production costs and times, thanks to the fact that it is preferably made in a single main piece.

These and other direct and complementary objects are achieved by the new orthopaedic device for the preventive and/or corrective and/or post- surgery treatment of hallux valgus, comprising a substantially flat body in an elongated shape, made of a substantially rigid material and suited to be placed on and constrained to the side the foot, precisely the big toe side, said body being provided with a first lateral front portion, substantially flat and rigid, suited to be rested on the part downstream of the hallux joint, a second lateral rear portion, substantially flat and rigid, suited to be rested upstream of the hallux joint, and an intermediate or central portion made of a flexible material in order to allow the relative rotation of said lateral portions, at least one the plane where they lie.

Therefore, constraining said new device to the side of the foot, with said central flexible portion at the level of the hallux joint, allows the flexion-extension movement of the hallux itself, while at the same time maintaining traction on the hallux itself.

In particular, according to the invention, said intermediate or central portion is in turn substantially in the shape of an accordion, for example comprising at least one or preferably one pair of substantially linear sections shaped as a V, said V-shaped sections having convergent vertices, in such a way that said intermediate or central portion is at least substantially in the shape of an X.

In the preferred embodiment of the invention, said substantially X-shaped central portion is curved, with its concave portion suited to be directed towards the foot and at the level of the projecting joint of the hallux.

The new device preferably comprises also a stiffening element or button substantially in the shape of a disc, having on one side one or more arched tabs suited to be connected to said sections of said central X-shaped portion, preferably on said concave portion. Said button substantially has the function to stiffen or strengthen said body, in order to prevent or at least limit the bending of the body crosswise with respect to the plane where the body itself lies.

Preferably, said button is at least partially made of a soft material and is suited to modify the stiffness and the overall thickness of the device according to the user's specific needs, for example according to the user's weight and to the degree of deviation of the big toe, to the presence or absence of wounds, etc.

Said lateral flat portions of said device furthermore comprise coupling means for straps suited to fasten the device to the foot, and in particular fastening straps suited to be wound around the foot upstream of the hallux joint and to be wound around the hallux downstream of the joint.

According to the invention, said body can be made in a single piece or said more flexible central portion can be produced separately and assembled on said lateral flat portions, for example through edge projections suited to be inserted in corresponding edge seats provided in the portion to be joined.

Said central portion is preferably made of a material different from that used for said lateral flat portions. For example, said lateral flat portions are made of a substantially rigid plastic material, while said central portion is made of a more flexible plastic material.

According to the solution with said central portion assembled on said lateral flat portions, said central portion can be interchangeable, too, so that it is possible to vary its degree of flexibility and therefore the characteristics of the orthopaedic device according to the needs.

Preferably, the new device comprises also one or more soft pads or inserts suited to be interposed between said body and the user's foot and between said button and the user's foot.

In the preferred solution, said new device comprises also at least one interposed anti friction layer suited to be removably or non-removably constrained to or in any case interposed between said device and the user's foot. In particular, said anti friction layer is positioned at least on said lateral rear portion.

Preferably, said at least one anti friction layer is completely or partially made of gel and/or fabric, for example felt or another type of fabric.

The new device, in particular said body and/or said button, is completely or partially made of a preferably plastic material and can be in one or more colours.

The characteristics of the new device are illustrated in greater detail in the following description with reference to the attached drawings which are enclosed hereto by way of non-limiting example.

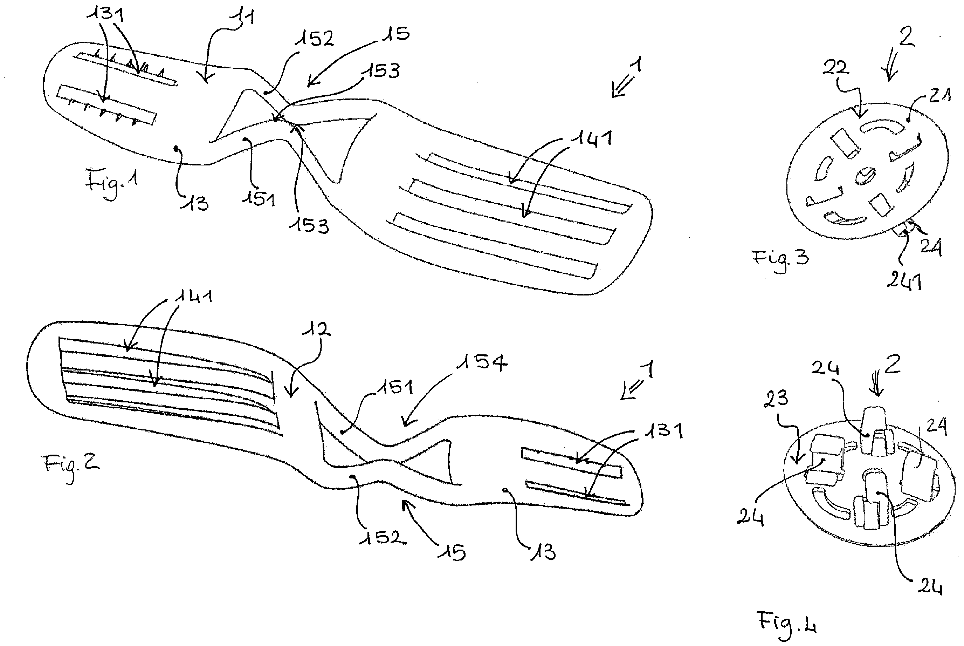

Figures 1 and 2 show two three-dimensional views of the body (1) of the new device (3), respectively representing the upper side (11), meaning the side intended to be opposite the foot, and the underside (12), intended to face the foot.
Figures 3 and 4 show the button (2) or stiffening element suited to be applied to said body (1), wherein said body (2) is represented in two three-dimensional views respectively showing the underside (22), intended to face the foot, and the upper side (23), intended to be facing towards said body (1) and provided with coupling elements or tabs (24) for connection to the body (1) itself.
Figure 5 shows a three-dimensional view of the new device when completely assembled (3), comprising said body (1) with the button (2) applied thereto.
Figure 6 shows a side view of the new device (3) when completely assembled, while
Figure 7 shows a view of the upper side (11) of said body (1) with the button (2) applied thereto.
Figures 8 and 9 respectively show two views of the new device (3) with the straps (4, 5) applied thereto, wherein said straps serve for fastening the device (3) to the user's foot.

The invention is an orthopaedic device (3) for the preventive and/or corrective and/or post-surgery treatment of hallux valgus, with minimized thickness and suited to be used day and night.

The new device (3) comprises a substantially flat and elongated body (1) suited to be positioned with one side, or underside (12) on the side of the foot, at the level of the hallux valgus either before or after surgery.

Said body (1) comprises:
- a substantially flat and rigid front lateral portion (13) suited to be rested downstream of the big toe joint,
- a substantially flat and rigid rear lateral portion (14) suited to be rested on the part upstream of the big toe joint,
- an intermediate or central portion (15) made of a flexible material in order to allow the relative rotation of said flat lateral portions (14, 13), at least on the plane where they lie.

In the preferred embodiment of the invention, said central portion (15) is made of a material different from that used for said lateral flat portions (14, 13), and in particular of a more flexible material compared to said lateral flat portions (14, 13).

In the preferred embodiment illustrated in the figures said body (1) is made in a single piece, but according to the invention said at least one intermediate or central portion (15) can also be removably or non-removably connected to said lateral flat portions (14, 13).

In a possible embodiment, said intermediate or central portion (15) is substantially in the shape of an accordion, for example comprising at least one or preferably one pair (151, 152) of substantially linear sections shaped as at least one V.

As can be seen in the figures, said sections (151, 152) are substantially linear and V-shaped with convergent vertices (153), in such a way that said intermediate or central portion (15) is substantially X-shaped.

Said substantially X-shaped central portion (15) is curved, defining on said underside (12) a concave portion (154) which is thus suited to face towards the foot, and therefore creating a concave portion suited to accommodate the bunion of the hallux joint.

The new device (3) comprises also a reinforcing element or button (2) with a substantially disc-shaped body (21) suited to be removably applied to said central portion (15) at the level of said concave portion (154).

For this purpose, said button (2) comprises, on one side (23), one or more coupling elements such as, for example, four arched tabs (24), meaning tabs with a folded end (241) that are suited to be connected in pairs to said two sections (151, 152) of said central X-shaped portion (15), at the level of said concave portion (154).

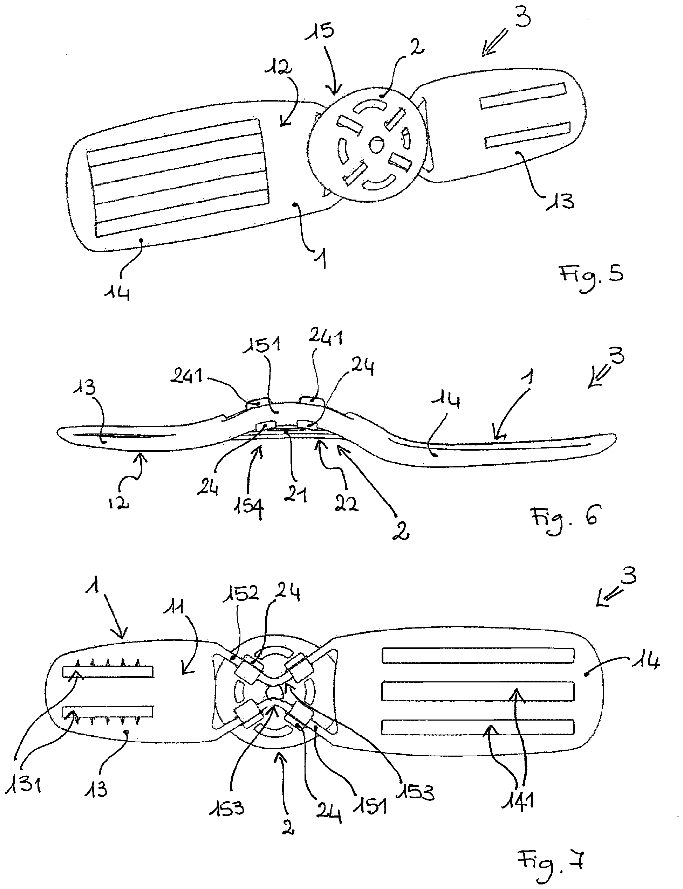

As shown in Figure 6, said button (2) is thus recessed in said concave portion (154) and has the function to stiffen or strengthen said body (1) in order to prevent or, in any case, limit its bending movement crosswise with respect to the plane where the body itself lies.

The opposite side (22) of said button (2) is intended to face the user's foot at the level of the joint and is preferably made of a soft material or covered or partially covered with a soft material, suitable for contact with the foot itself.

Analogously, on said underside (12) of said body (1), at the level of said lateral portions (13, 14), there are soft inserts or elements suited to be interposed between the body (1) itself and the foot, in order to dampen the contact with the foot.

Said lateral flat portions (13, 14) of said body (1) furthermore comprise coupling means for straps or belts (4, 5) suited to fasten the device to the foot.

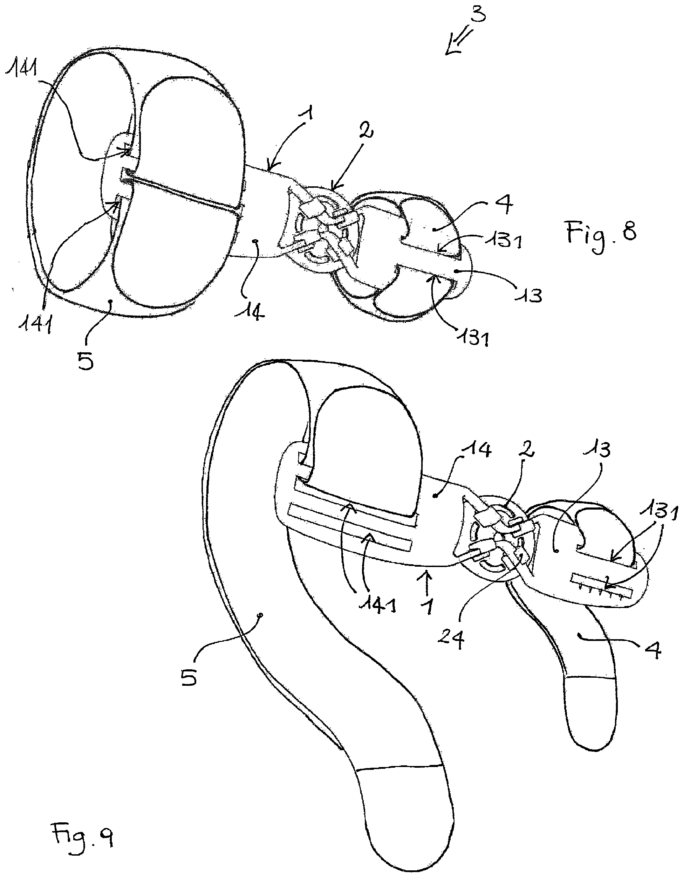

Said lateral portions (13, 14), for example, are provided with slots or slotted holes (131, 141) suitable for the insertion of said belts or straps (4, 5), which then must be wound (see Figure 8) around the user's foot, in a removable manner.

In particular, said front lateral portion (13) is constrained to the big toe, downstream of the joint, by means of at least one of said straps (4), while said rear lateral portion (14) is constrained to the foot, upstream of the big toe joint, by means of at least one of said straps (5).

Therefore, with reference to the above description and the attached drawings, the following claims are expressed.

## Claims

1. Orthopaedic device (3) for hallux valgus, said device comprises a substantially flat and elongated body (1) suited to be constrained in such a way as to be positioned with one side (12), or underside, beside the foot, at the level of the hallux, wherein said body (1) in turn comprises:
• a substantially flat and rigid front lateral portion (13) suited to be rested downstream of the hallux joint,
• a substantially flat and rigid rear lateral portion (14) suited to be rested on the part upstream of the hallux joint,
**characterized in that** said body further comprises
• an intermediate or central portion (15) suited to be substantially rested at the level of the hallux joint and made of a flexible material,
and wherein said flexible central portion (15) allows the relative rotation of said flat lateral portions (14, 13), at least on the plane on which they lie, while traction on the hallux is maintained.

2. Orthopaedic device (3) according to claim 1, **characterized in that** said intermediate or central portion (15) is substantially configured as an accordion.

3. Orthopaedic device (3) according to claim 2, **characterized in that** said intermediate or central portion (15) comprises at least one pair (151, 152) of substantially linear sections configured as at least one V with converging vertices (153), in such a way that said intermediate or central portion (15) is substantially X-shaped.

4. Orthopaedic device (3) according to claim 2 or 3, **characterized in that** said central portion (15) is curved and defines a concave part (154) facing towards said underside (12) and therefore suited to face towards the foot, thus creating a recess suited to house the bunion of the big toe joint.

5. Orthopaedic device (3) according to the preceding claims, **characterized in that** it comprises at least one reinforcing element or button (2) having a substantially disc-shaped body (21) suited to be applied to said central portion (15) in a removable manner, at the level of said concave part (154), and having the function to selectively stiffen said body (1) in order to prevent or in any case limit its bending movement in a direction that is transverse to the plane on which the body lies.

6. Orthopaedic device (3) according to claim 5, **characterized in that** said button (2) comprises, on the side (23) facing towards said body (1), one or more coupling elements or arched tabs (24) suited to be coupled with said central portion (15) in a removable manner, at the level of said concave part (154).

7. Orthopaedic device (3) according to claim 5 or 6, **characterized in that** said button (2) is interchangeable so that it is possible to vary the stiffness of said body (1).

8. Orthopaedic device (3) according to the preceding claims, **characterized in that** said central portion (15) is made of a material that is different from the material with which said flat lateral portions (14, 13) are made, and in particular of a material that is more flexible than that used for said flat lateral portions (14, 13).

9. Orthopaedic device (3) according to one or more of the claims from 1 to 8, **characterized in that** said body (1) is made in a single piece.

10. Orthopaedic device (3) according to one or more of the claims from 1 to 8, **characterized in that** said lateral portions (13, 14) and said central portion (15) of said body (1) are separate and interchangeable parts that can be constrained to one another in a removable manner.

11. Orthopaedic device (3) according to the preceding claims, **characterized in that** it comprises one or more bearings or inserts or layers suited to be interposed between the entirety or part of said body (1) and the patient's foot and/or between said button (2) and the patient's foot.

12. Orthopaedic device (3) according to the preceding claims, **characterized in that** said flat lateral parts (13, 14) of said body (1) comprise coupling means for belts or straps (4, 5) suited to be wound around the foot and the hallux in order to fix the device to the foot.

## Patentansprüche

1. Orthopädische Vorrichtung (3) für Hallux Valgus, wobei die besagte Vorrichtung einen im Wesentlichen flachen und länglichen Körper (1) umfasst, der geeignet ist, so fixiert zu werden, dass er mit einer Seite (12) oder mit der Unterseite neben dem Fuß positioniert wird, auf Ebene des Großzehs, wobei der besagte Körper (1) seinerseits Folgendes umfasst:
• einen im Wesentlichen flachen und steifen, seitlichen Vorderabschnitt (13), dazu geeignet, nach dem Großzehgelenk aufgelegt zu werden,
• einen im Wesentlichen flachen und steifen, seitlichen Hinterabschnitt (14), dazu geeignet, auf den Teil vor dem Großzehgelenk aufgelegt zu werden, **dadurch gekennzeichnet, dass** der besagte Körper des Weiteren Folgendes umfasst:
• einen Zwischen- oder Mittelabschnitt (15), dazu geeignet, im Wesentlichen auf Ebene des Großzehgelenks aufgelegt zu werden, und aus einem flexiblen Material bestehend,
und wobei der besagte flexible Mittelabschnitt (15) wenigstens auf der Ebene, auf der sie liegen, die relative Drehung der besagten flachen Seitenabschnitte (14, 13) erlaubt, während die Traktion am Großzeh erhalten bleibt.

2. Orthopädische Vorrichtung (3) nach Patentanspruch 1, **dadurch gekennzeichnet, dass** der besagte Zwischen- oder Mittelabschnitt (15) im Wesentlichen wie ein Akkordeon konfiguriert ist.

3. Orthopädische Vorrichtung (3) nach Patentanspruch 2, **dadurch gekennzeichnet, dass** der besagte Zwischen- oder Mittelabschnitt (15) wenigstens ein Paar (151, 152) im Wesentlichen geradliniger Bereiche umfasst, die als wenigstens ein V mit konvergierenden Scheiteln (153) konfiguriert sind, so dass der besagte Zwischen- oder Mittelabschnitt (15) im Wesentlichen X-förmig ist.

4. Orthopädische Vorrichtung (3) nach Patentanspruch 2 oder 3, **dadurch gekennzeichnet, dass** der besagte Mittelabschnitt (15) gewölbt ist und einen konkaven Teil (154) definiert, der zu der besagten Unterseite (12) gerichtet ist und daher geeignet ist, zum Fuß gerichtet zu sein und somit eine Vertiefung zu bilden, die geeignet ist, den Ballen des Großzehgelenks aufzunehmen.

5. Orthopädische Vorrichtung (3) nach vorstehenden Patentansprüchen, **dadurch gekennzeichnet, dass** sie wenigstens ein Verstärkungselement oder einen Knopf (2) mit einem im Wesentlichen scheibenförmigen Körper (21) umfasst, der geeignet ist, auf Ebene des besagten konkaven Teils (154) abnehmbar an dem besagten Mittelabschnitt (15) angebracht zu werden, und der dazu dient, den besagten Körper (1) selektiv zu versteifen, um seine Beugebewegung in eine quer zu der Ebene, auf welcher der Körper aufliegt, verlaufende Richtung zu vermeiden oder in jedem Fall zu begrenzen.

6. Orthopädische Vorrichtung (3) nach Patentanspruch 5, **dadurch gekennzeichnet, dass** der besagte Knopf (2) auf der zu dem besagten Körper (1) gerichteten Seite (23) ein oder mehrere Kupplungselemente oder gebogene Laschen (24) umfasst, die dazu geeignet sind, auf Ebene des besagten konkaven Teils (154) abnehmbar mit dem besagten Mittelabschnitt (15) verbunden zu werden.

7. Orthopädische Vorrichtung (3) nach Patentanspruch 5 oder 6, **dadurch gekennzeichnet, dass** der besagte Knopf (2) austauschbar ist, so dass die Steifigkeit des besagten Körpers (1) veränderbar ist.

8. Orthopädische Vorrichtung (3) nach vorstehenden Patentansprüchen, **dadurch gekennzeichnet, dass** der besagte Mittelabschnitt (15) aus einem Material besteht, das sich von dem Material unterscheidet, aus dem die besagten flachen Seitenabschnitte (14, 13) gefertigt sind, und insbesondere aus einem Material, das flexibler ist als jenes, das für die besagten flachen Seitenabschnitte (14, 13) verwendet wird.

9. Orthopädische Vorrichtung (3) nach einem oder mehreren der Patentansprüche von 1 bis 8, **dadurch gekennzeichnet, dass** der besagte Körper (1) aus einem Stück gefertigt ist.

10. Orthopädische Vorrichtung (3) nach einem oder mehreren der Patentansprüche von 1 bis 8, **dadurch gekennzeichnet, dass** die besagten Seitenabschnitte (13, 14) und der besagte Mittelabschnitt (15) des besagten Körpers (1) separate und austauschbare Teile sind, die abnehmbar miteinander verbunden werden können.

11. Orthopädische Vorrichtung (3) nach vorstehenden Patentansprüchen, **dadurch gekennzeichnet, dass** sie ein oder mehrere Lager oder Einsätze oder Lagen umfasst, die geeignet sind, zwischen der Gesamtheit oder einem Teil des besagten Körpers (1) und den Fuß des Patienten und/oder zwischen dem besagten Knopf (2) und dem Fuß des Patienten eingefügt zu werden.

12. Orthopädische Vorrichtung (3) nach vorstehenden Patentansprüchen, **dadurch gekennzeichnet, dass** die besagten flachen Seitenteile (13, 14) des besagten Körpers (1) Kupplungsmittel für Riemen oder Bänder (4, 5) umfassen, die dazu geeignet sind, um den Fuß und den Großzeh gewickelt zu werden, um die Vorrichtung am Fuß zu fixieren.

## Revendications

1. Dispositif orthopédique (3) pour l'hallux valgus, ledit dispositif comprenant un corps essentiellement plat et allongé (1) indiqué pour être bloqué de manière à être positionné avec un côté (12), ou le dessous, à côté du pied, à hauteur de l'hallux, où ledit corps (1) comprend à son tour :
• une portion latérale avant essentiellement plate et rigide (13) indiquée pour être appuyée contre la partie en aval de l'articulation de l'hallux,
• une portion latérale arrière essentiellement plate et rigide (14) indiquée pour être appuyée contre la partie en amont de l'articulation de l'hallux,
**caractérisé en ce que** ledit corps comprend en outre
• une portion intermédiaire ou centrale (15) indiquée pour être appuyée essentiellement à hauteur de l'articulation de l'hallux et réalisée en un matériau flexible,
et où ladite portion centrale flexible (15) permet la rotation relative desdites portions latérales (14, 13), au moins sur leur plan sur lequel elles reposent, alors que la traction sur l'hallux est maintenue.

2. Dispositif orthopédique (3) selon la revendication 1, **caractérisé en ce que** ladite portion intermédiaire ou centrale (15) est essentiellement configurée comme un accordéon.

3. Dispositif orthopédique (3) selon la revendication 2, **caractérisé en ce que** ladite portion intermédiaire ou centrale (15) comprend au moins une paire (151, 152) de sections essentiellement linéaires configurées au moins en V, avec sommets convergents (153) de façon à ce que ladite portion intermédiaire ou centrale (15) résulte essentiellement en X.

4. Dispositif orthopédique (3) selon la revendication 2 ou 3, **caractérisé en ce que** ladite partie centrale (15) est courbe et définit une partie concave (154) tournée vers ledit dessous (12) et donc indiquée pour être tournée vers le pied, en réalisant ainsi une cavité apte à loger l'hallux valgus de l'articulation du gros orteil.

5. Dispositif orthopédique (3) selon les revendications précédentes, **caractérisé en ce qu'**il comprend au moins un élément de renfort ou bouton (2) ayant un corps (21) essentiellement en forme de disque indiqué pour être appliqué de manière amovible à ladite portion centrale (15), à hauteur de ladite partie concave (154), et ayant la fonction de raidir sélectivement ledit corps (1) afin d'empêcher, ou de toute façon limiter, son mouvement de flexion dans la direction transversale au plan sur lequel le corps repose.

6. Dispositif orthopédique (3) selon la revendication 5, **caractérisé en ce que** ledit bouton (2) comprend, sur le côté (23) tourné vers ledit corps (1), un ou plusieurs éléments d'accouplement ou ailettes en arc (24) aptes à être accouplés de manière amovible à ladite portion centrale (15), à hauteur de ladite partie concave (154).

7. Dispositif orthopédique (3) selon la revendication 5 ou 6, **caractérisé en ce que** ledit bouton (2) est interchangeable de manière à ce qu'il est possible de varier la rigidité dudit corps (1).

8. Dispositif orthopédique (3) selon les revendications précédentes, **caractérisé en ce que** ladite portion centrale (15) est réalisée en un matériau différent par rapport au matériau avec lequel lesdites portions latérales planes (14, 13) sont réalisées, et en particulier en un matériau plus flexible par rapport à celui utilisé pour lesdites portions latérales planes (14, 13).

9. Dispositif orthopédique (3) selon l'une ou plusieurs desdites revendications de 1 à 8, **caractérisé en ce que** ledit corps (1) est réalisé en une seule pièce.

10. Dispositif orthopédique (3) selon l'une ou plusieurs des revendications de 1 à 8, **caractérisé en ce que** lesdites portions latérales (13, 14) et ladite portion centrale (15) dudit corps (1) sont réalisées en parties séparées et interchangeables qui peuvent être bloquées entre elles de manière amovible.

11. Dispositif orthopédique (3) selon les revendications précédentes, **caractérisé en ce qu'**il comprend un ou plusieurs roulements ou insertions ou couches indiqués pour être interposés entre la totalité dudit corps (1) ou une partie de celui-ci et le pied du patient et/ou entre ledit bouton (2) et le pied du patient.

12. Dispositif orthopédique (3) selon les revendications précédentes, **caractérisé en ce que** lesdites parties latérales planes (13, 14) dudit corps (1) comprennent des moyens d'accouplement pour courroies ou sangles (4, 5) aptes à être enroulées autour du pied et de l'hallux de manière à fixer le dispositif au pied.
